# EUROPEAN PATENT APPLICATION

(11) **EP 0 561 407 A1**
(43) Date of publication of application: **22.09.1993**
(21) Application number: 93104485.3
(22) Date of filing: 18.03.1993
(51) Int. Cl.: A61L 15/07

(54) **Method for obtaining a traumatological plaster cast with waterproof and resistance qualities**

(30) Priority: 18.03.1992 ES 9200591
(71) Applicant: GRUPO KENNEY (SERVICE AND FINANCIAL CONSULTING, S.L.), E-14008 Cordoba (ES)
(72) Inventor: Herrera Molina, José Luis, E-14008 Cordoba (ES)
(74) Representative: Patentanwälte Dr. Solf & Zapf

(57) **Abstract**

The invention relates to a method for obtaining a traumatological plaster cast with waterproof and resistance qualities characterized by adding the following constituents to a receptacle: an indeterminate volume made up of 96% warm water, 7,65 pH value, a dry residue of 696% mg, 43% mg Na sodium, 7,30% mg K potassium, 8,02% mg Ca, 1,20% mg Mg, 0,40% mg Am, 580% mg. nitrate, 0,53% mg nitrite, 25,6% mg sulphate, < 0,004% mg cyanide, < 0,05% mg phosphorus, < 0,05% mg iron, < 0,06% mg manganese, < 0,05% mg copper and < 0,10% mg of aluminium, whereby the organic and inorganic elements may consist of a mixture of powder and liquid.

## Description

The invention relates to a method for obtaining a traumatological plaster cast with waterproof and resistance qualities and introduces a fundamental innovation absent in foregoing procedures to obtain a traumatological plaster cast.

### PRIOR ART

Gypsum or dried calcium sulphate is the most common of all sulphates. It principally comes from the evaporation of salt water and its crystals are found in very varied forms. Plaster of Paris is fine gypsum obtained by heating the crude gypsum to 120°C. The use of the plaster cast is a commonly employed method in the immobilization of parts of the body presenting bone injuries and fractures in particular. Although there is evidence that this therapeutic method was used way back in ancient times, it was only from 1850 onwards tht the present type of plaster, consisting of a bandage impregnated with gypsum in a uniform layer, was employed.

Plaster casts can be applied using many different techniques. The most common method consists of binding in a circular direction using a gypsum bandage previously soaked in warm water. Several bandages are enfolded in quick succession around the part of the body to be immobilized and the cast is moulded into shape. After a few minutes, the plaster "sets" and becomes hard. The plaster is left in situ 20, 30, or 40 days, and sometimes longer, according to orthopaedic instruction, until callus formation occurs at the fracture site.

Finally, the cast is removed using a suitable method. Different methods of applying therapeutic cast have lead to slight differences to the general procedure described above.

In all cases, however, the procedures have a common disadvantage: they do not resist continued contact with water since the gypsum cast, originally formed by evaporation and dehydration, loses its principal characteristics and advantages. The consistency and hardness of the present plaster casts deteriorate to such an extent that they cannot be brought into contact with water at all.

In addition, another negative characteristic of plaster casts used in present techniques is that they have little resistance to impacts; they break totally or partially comparatively easily.

Object of the present invention is to provide a plaster cast with waterproof and resistance qualities which does not exhibit the above-mentioned drawbacks.

### DESCRIPTION OF THE INVENTION

The present invention provides a method for obtaining a traumatological plaster cast with waterproof and resistance qualities, characterized by adding the following constituents to a receptacle: an indeterminate volume made up of 96% warm water, 7,65 pH value, a dry residue of 696% mg, 43% mg Na sodium, 7,30% mg K potassium, 8,02% mg Ca, 1,20% mg Mg, 0,40% mg Am, 580% mg nitrate, 0,53% mg nitrite, 25,6% mg sulphate, < 0,004% mg cyanide, < 0,05% mg phosphorous, < 0.05% mg iron, < 0,06% mg manganese, < 0,05% mg copper and < 0,10% mg of aluminium, whereby the organic and inorganic elements may consist of a mixture of powder and liquid.

A preferred method is characterized in that the powder gypsum is added in a proportional quantity to the mixture of warm water and organic and inorganic products; the resulting mixture takes form; circular bandages are then introduced in the said mixture, the soaked bandages are then squeezed out and applied, still wet, around the injured limb or part of the body.

The procedure to obtain a plaster cast with excellent waterproof and resistance qualities introduces the new and considerable advantage that the plaster can get wet; it is completely resistant to water in general, such as rainwater, showers and even baths. The cast, when brought into contact with water, will not dissolve, break up or shed plaster particles. The procedure is based on traditional methods for obtaining gypsum and plaster for traumatological uses, with the addition of special complementary products which modify the molecular structure of the gypsums and plasters, transforming the latter into a waterproof material which is more resistant and has a harder surface. These special products may come either in liquid form, in which case it would be added to the impregnated preparation of the plaster bandage, or they may come in solid form, which would be ground into the gypsum powder.

This procedure makes the cast more resistant and harder which in turn enables the cast to be made more thinly and means that less bandage and impregnated gypsum is needed. A secondary advantage of the invention is that the results of the cast are equal to those of casts used at present and that application remains simple and hardly differs at all. This plaster is especially intended for use in both medical and veterinary traumatological casts.

A further embodiment comprises a method for obtaining a traumatological plaster cast with waterproof and resistance qualities, characterized in that 1.25% hydrogene peroxide are added to a container having a certain value and being filled up to 96% with warm water and admixing thereafter 0.25% iodine, 1.25% acetylsalicylic acid and 1.25% gelatine, whereby the admixed products can be provided in form of an organic and anorganic admixture in powder or liquid form; and that the gypsum is added to the mixture consisting of warm water and the organic and anorganic products in powdery form in suitable proportions while providing a homogeneous mixture and that the bandages of the plaster cast are immersed therein and after dripping are bandaged around the fractured limb or part of the body.

### PROCEDURE TO OBTAIN THE PLASTER

The fine gypsum or plaster used in the procedure in this invention is the same as that used in traditional traumatological applications in human and veterinary medicine. Thus, the first stage of preparation in the procedure in this invention coincides with the traditional preparation with which we are familiar. The difference with this novel innovation is seen when the bandage is to be impregnated in gypsum. The latter is mixed, using a suitable procedure and perfectly calculated quantities, with special products consisting of inorganic elements which may either be liquid, dissolved in water and already mixed in the warm water in which the gypsum bandage is to be submerged for subsequent circular bandaging, or solid, in the form of a powder which would be previously mixed with the gypsum or plaster and subsequently impregnated in the bandage.

The gypsum bandages or strips containing the product are then applied as normal, enfolding the part of the body to be immobilized without delay. At this moment a chemical reaction commences which prevents crystalization of the plaster at the same time as it caused the majority of the water retained in the bandage to be expelled.

These reactions bring about a considerable change in the molecular structure of the gypsum or plaster. The material acquires other properties, including a harder outer surface and a different moisture-thermal relation which prevents the continual exchange of humidity with the atmosphere and materials with which it may be in contact.

When the plaster cast that has been prepared this product has set, it becomes waterproof and highly resistant. The plaster wearer or patient therefore enjoys two great advantages which are as follows:
1) The wearer will be able to attend to his hygiene needs as a perfectly healthy person does, because he will be able to shower or have baths, submerging the plastered limb or affected part totally freely, without worrying that when the plaster comes into contact with the water it may start to dissolve, break up or shed particles, which indeed has been the case up until now with the traumatological casts used a present and described above.
2) Since the plaster cast will be thinner than normal, it will obviously weigh less than the traditional cast that has been used up until now. The plastered limb or affected part will therefore no longer suffer a double immobilization, i.e., the therapeutic immobilization which is the fundamental purpose of the cast, and the negative subsidiary immobilization brought about by the weight or ballast which up until now has caused tremendous limitation in the wearer's mobilization in general.

These two advantages are completely new; they imply enormous progress in the field of traumatology and medicine in general, for both therapeutical and traumatological interests and for the actual comfort, or rather, decreased discomfort and limitations of the person treated with a therapeutic traumatological plaster cast procedure. This invention of a procedure for obtaining a traumatological plaster cast with waterproof and resistance qualities represents an absolute innovation in the field of medicine considering that at present there is no procedure that provides the qualities and innovations contemplated in this invention.

## Claims

1. Method for obtaining a traumatological plaster cast with waterproof and resistance qualities, **characterized by** adding the following constituents to a receptacle: an indeterminate volume made up of 96% warm water, 7,65 pH value, a dry residue of 696% mg, 43% mg Na sodium, 7,30% mg K potassium, 8,02% mg Ca, 1,20% mg Mg, 0,40% mg Am, 580% mg nitrate, 0,53% mg nitrite, 25,6% mg sulphate, < 0,004% mg cyanide, < 0,05% mg phosphorus, < 0,05% mg iron, < 0,06% mg manganese, < 0,05% mg copper and < 0,10% mg of aluminium, whereby the organic and inorganic elements may consist of a mixture of powder and liquid.

2. Method for obtaining a traumatological plaster cast with waterproof and resistance qualities according to claim 1, **characterized in that** the powder gypsum is added in a proportional quantity to the mixture of warm water and organic and inorganic products; the resulting mixture takes form; circular bandages are then introduced in the said mixture; the soaked bandages are then squeezed out and applied, still wet, around the injured limb or part of the body.
